(19) Europäisches Patentamt
European Patent Office
Office européen des brevets

(11) Veröffentlichungsnummer: **0 666 287 A2**

(12) **EUROPÄISCHE PATENTANMELDUNG**

(21) Anmeldenummer: **95101121.2**

(22) Anmeldetag: **27.01.95**

(51) Int. Cl.[6]: **C09B 29/30**, C07C 311/46, C09B 44/00

(30) Priorität: **07.02.94 DE 4403667**

(43) Veröffentlichungstag der Anmeldung: **09.08.95 Patentblatt 95/32**

(84) Benannte Vertragsstaaten: **AT CH DE FR GB IT LI SE**

(71) Anmelder: **BASF AKTIENGESELLSCHAFT**
**D-67056 Ludwigshafen (DE)**

(72) Erfinder: **Schlösser, Ulrike, Dr.**
**In den Weihergärten 25**
**D-67071 Ludwigshafen (DE)**
Erfinder: **Mayer, Udo, Dr.**
**Max Slevogt Strasse 27**
**D-67227 Frankenthal (DE)**

(54) **Basische Farbstoffe auf Basis von Amiden der 1-Hydroxy-6-aminonaphtalin-3-sulfonsäure sowie deren Zwischenprodukte.**

(57) Die vorliegende Erfindung betrifft neue Farbstoffe der Formel

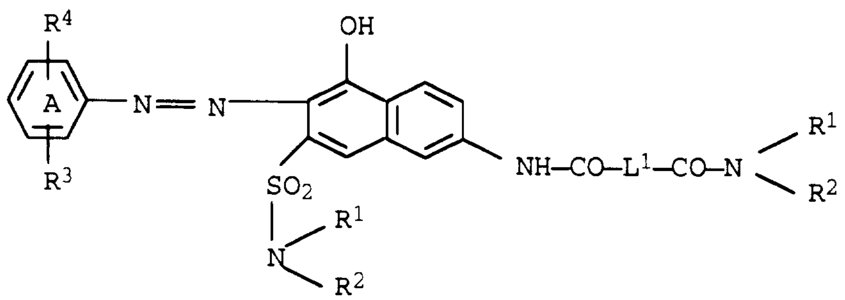

in der
der Ring A benzoanelliert sein kann,

- $L^1$ $C_2$-$C_3$-Alkylen, das gegebenenfalls durch Chlor substituiert ist, Vinylen oder 1,2-Phenylen,
- $R^1$ und $R^2$ gegebenenfalls substituiertes $C_1$-$C_{13}$-Alkyl, gegebenenfalls substituiertes $C_5$-$C_7$-Cycloalkyl oder gegebenenfalls substituiertes Piperidinyl oder $R^1$ auch Wasserstoff oder $R^1$ und $R^2$ zusammen mit dem sie verbindenden Stickstoffatom einen heterocyclischen Rest,
- $R^3$ Wasserstoff, $C_1$-$C_4$-Alkyl, $C_1$-$C_4$-Alkoxy, $C_2$-$C_5$-Alkanoylamino, Halogen, Nitro, Cyano, gegebenenfalls substituiertes Phenylazo, $C_2$-$C_4$-Alkanoylamino, das durch eine quaternierbare oder quartäre Gruppe substituiert ist, oder $C_2$-$C_4$-Alkoxy, $C_2$-$C_4$-Alkoxycarbonyl oder $C_2$-$C_4$-Alkoxycarbamoyl, das jeweils durch eine quaternierbare oder quartäre Gruppe substituiert ist, und
- $R^4$ Wasserstoff, $C_1$-$C_4$-Alkyl, $C_1$-$C_4$-Alkoxy, Halogen, Nitro oder Cyano bedeuten,

mit der Maßgabe, daß mindestens ein quaternierbares oder quartäres Stickstoffatom im Molekül vorhanden ist, deren Verwendung zum Färben oder Bedrucken von polymerem Material sowie neue Zwischenprodukte.

EP 0 666 287 A2

Die vorliegende Erfindung betrifft neue Farbstoffe der Formel I

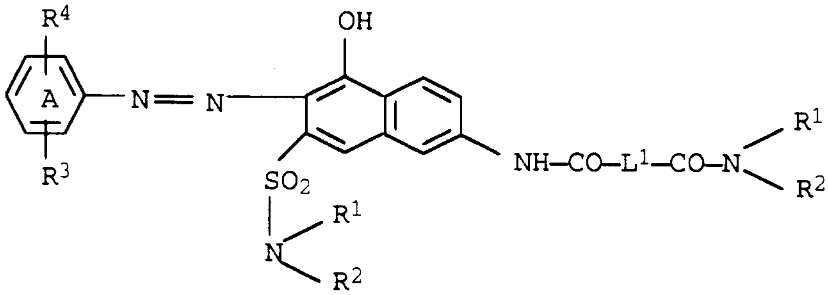

(I),

in der
der Ring A benzoanelliert sein kann,

$L^1$ $C_2$-$C_3$-Alkylen, der gegebenenfalls durch Chlor substituiert ist, Vinylen oder 1,2-Phenylen,

$R^1$ und $R^2$ gleich oder verschieden sind und unabhängig voneinander jeweils $C_1$-$C_{13}$-Alkyl, das gegebenenfalls durch 1 bis 4 Sauerstoffatome in Etherfunktion oder 1 bis 4 Iminogruppen, die durch $C_1$-$C_4$-Alkyl oder $\omega$-Hydroxy-$C_1$-$C_8$-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder ungesättigten heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes $C_5$-$C_7$-Cycloalkyl substituiert sein kann, gegebenenfalls substituiertes Phenyl, gegebenenfalls substituiertes $C_5$-$C_7$-Cycloalkyl oder gegebenenfalls durch $C_1$-$C_4$-Alkyl substituiertes Piperidinyl oder $R^1$ auch Wasserstoff oder $R^1$ und $R^2$ zusammen mit dem sie verbindenden Stickstoffatom einen 5- oder 6-gliedrigen gesättigten heterocyclischen Rest, der gegebenenfalls ein weiteres Heteroatom, ausgewählt aus der Gruppe, bestehend aus Sauerstoff und Schwefel, aufweist,

$R^3$ Wasserstoff, $C_1$-$C_4$-Alkyl, $C_1$-$C_4$-Alkoxy, $C_2$-$C_5$-Alkanoylamino, Halogen, Nitro, Cyano, gegebenenfalls durch $C_1$-$C_4$-Alkyl substituiertes Phenylazo oder einen Rest der Formel

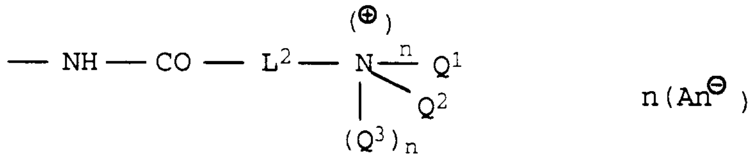,

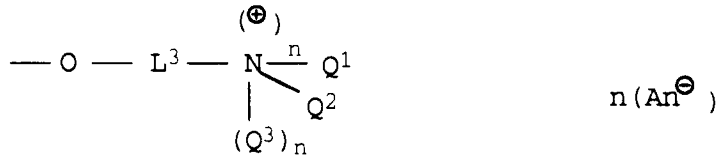,

oder

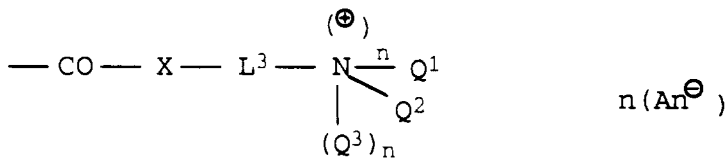,

worin X für Sauerstoff oder Imino,

$L^2$ für $C_1$-$C_3$-Alkylen,

$L^3$ für $C_2$-$C_4$-Alkylen,

n für 0 oder 1,

$Q^1$, $Q^2$, $Q^3$ gleich oder verschieden sind und unabhängig voneinander jeweils für Wasserstoff oder gegebenenfalls durch Hydroxy substituiertes $C_1$-$C_8$-Alkyl, das durch 1 bis 3 Sauerstoffatome in Etherfunktion oder 1 bis 3 Iminogruppen, die durch $C_1$-$C_4$-Alkyl oder $\omega$-Hydroxy-$C_1$-$C_8$-alkyl, das gegebenenfalls durch 1 bis 3 Sauerstoffatome in Etherfunktion unterbrochen ist, substituiert sein können, unterbrochen sein kann, oder $Q^1$ und $Q^2$ zusammen mit dem sie verbindenden Stickstoffatom für einen 5- oder 6-gliedrigen gesättigten oder ungesättigten heterocyclischen Rest, der gegebenenfalls ein weiteres Heteroatom, ausgewählt aus der Gruppe, bestehend aus Stickstoff, Sauerstoff und Schwefel, aufweist, und

$An^{\ominus}$ für das Äquivalent eines Anions stehen, und

$R^4$ Wasserstoff, $C_1$-$C_4$-Alkyl, $C_1$-$C_4$-Alkoxy, Halogen, Nitro oder Cyano bedeuten,

mit der Maßgabe, daß mindestens ein quaternierbares oder quartäres Stickstoffatom im Molekül vorhanden ist, deren Verwendung zum Färben oder Bedrucken von polymerem Material sowie deren Zwischenprodukte.

Aufgabe der vorliegenden Erfindung war es, neue basische Azofarbstoffe mit einer Kupplungskomponente aus der Reihe Amide der I-Säure(1-Hydroxy-6-aminonaphthalin-3-sulfonsäure) bereit zustellen. Die neuen Farbstoffe sollten sich in vorteilhafter Weise zum Färben oder Bedrucken von polymerem Material, insbesondere von Papier eignen. Die erzielten Färbungen sollten gute Gebrauchseigenschaften aufweisen.

Demgemäß wurden die eingangs näher bezeichneten Farbstoffe der Formel I gefunden.

Alle in der obengenannten Formel auftretenden Alkyl- oder Alkylengruppen können sowohl geradkettig als auch verzweigt sein.

Wenn in der obengenannten Formel substituierte Phenylgruppen auftreten, so können als Substituenten z.B. $C_1$-$C_4$-Alkyl, $C_1$-$C_4$-Alkoxy oder Halogen, dabei insbesondere Chlor oder Brom, in Betracht kommen. Die Phenylgruppen weisen dann in der Regel 1 bis 3 Substituenten auf.

Wenn in der obengenannten Formel substituierte $C_5$-$C_7$-Cycloalkylgruppen auftreten, so können als Substituenten z.B. $C_1$-$C_4$-Alkyl oder $C_1$-$C_4$-Aminoalkyl in Betracht kommen. Die Cycloalkylgruppen weisen dann in der Regel 1 bis 3 Alkyl- und/oder eine Aminoalkylgruppe auf.

Wenn in der obengenannten Formel durch $C_1$-$C_4$-Alkyl substituiertes Piperidinyl auftritt, so weist es in der Regel 1 bis 4 $C_1$-$C_4$-Alkylgruppen auf, dabei insbesondere Methylgruppen, wobei substituiertes Piperidin-4-yl bevorzugt ist.

Wenn die Reste $R^1$ oder $R^2$ $C_1$-$C_{13}$-Alkyl bedeuten, das durch einen 5- oder 6-gliedrigen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, substituiert ist, so können als Substituenten gesättigte oder ungesättigte Reste, wie Pyrrolidinyl, Piperidinyl, Morpholinyl, Thiomorpholinyl, Piperazinyl, N-($C_1$-$C_4$-Alkyl)piperazinyl, Pyrrolyl, Pyrazolyl, Imidazolyl, Oxazolyl, Isooxazolyl, Thiazolyl oder Isothiazolyl, in Betracht kommen.

Diese Reste sind auch zu nennen, wenn $Q^1$ und $Q^2$ zusammen mit dem sie verbindenden Stickstoffatom einen 5- oder 6-gliedrigen heterocyclischen Rest bedeuten.

Wenn $R^1$ und $R^2$ zusammen mit dem sie verbindenden Stickstoffatom einen 5- oder 6-gliedrigen gesättigten heterocyclischen Rest, der gegebenenfalls ein weiteres Heteroatom, ausgewählt aus der Gruppe, bestehend aus Stickstoff, Sauerstoff und Schwefel, enthält, bedeuten, so können z.B. Pyrrolidinyl, Piperidinyl, Morpholinyl, Thiomorpholinyl, Piperazinyl oder N-($C_1$-$C_4$-Alkyl)piperazinyl als solche Reste in Betracht kommen.

Wenn in der obengenannten Formel substituierte Alkylreste auftreten, so sind diese in der Regel ein- oder zweifach substituiert.

Reste $R^1$, $R^2$, $R^3$, $R^4$, $Q^1$, $Q^2$ und $Q^3$ sind z.B. Methyl, Ethyl, Propyl, Isopropyl, Butyl, Isobutyl, sec-Butyl oder tert-Butyl.

Reste $R^1$, $R^2$, $Q^1$, $Q^2$, und $Q^3$ sind weiterhin z.B. Pentyl, Isopentyl, Neopentyl, tert-Pentyl, Hexyl, 2-Methylpentyl, Heptyl, Octyl, 2-Ethylhexyl, Isooctyl, 2-Methoxyethyl, 2-Ethoxyethyl, 2-Propoxethyl, 2-Isoproporyethyl, 2-Butoxethyl, 2- oder 3-Methoxpropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxyoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 2-Hydroxyethyl, 2- oder 3-Hydroxyethyl, 2- oder 4-Hydroxybutyl, 3-Aza-3-methylbutyl, 4-Aza-4-methylpentyl, 3-Aza-3-ethylpentyl oder 4-Aza-4-ethylhexyl.

Reste $R^1$ und $R^2$ sind weiterhin z.B. Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Benzyl, 1- oder 2-Phenylethyl, Phenyl, 2-, 3- oder 4-Methylphenyl, 2,4-Dimethylphenyl, 2-, 3- oder 4-Methoxyphenyl, 2,4-Dimethoxyphenyl, 2-, 3- oder 4-Chlorphenyl, 2,4-Dichlorphenyl, 2-Aminoethyl, 2- oder 3-Aminopropyl, 2- oder 4-Aminobutyl, 5-Aminopentyl, 6-Aminohexyl, 7-Aminoheptyl, 8-Aminooctyl, 5-Amino-3-azapentyl, 6-Amino-3-azahexyl, 6-Amino-4-azahexyl, 7-Amino-4-azaheptyl, 8-Amino-3, 6-diazaoctyl, 3-Aminoprop-2-yl, 8-Amino-4-(2-aminoethyl)octyl, 2-(Pyrrolidin-1-yl)ethyl, 2- oder 3-(Pyrrolidin-1-yl)propyl, 2-(Piperidin-1-yl)ethyl, 2- oder 3-(Piperidin-1-yl)propyl, 2-(Morpholin-4-yl)ethyl, 2- oder 3-(Morpholin-4-yl)propyl, 2-(Piperazin-1-yl)ethyl, 2- oder 3-(Piperazin-1-yl)propyl, 2-(4-Methylpiperazin-1-yl)-ethyl, 2- oder 3-(4-Methylpiperazin-1-yl)propyl, 2-(Imidazol-1-yl)ethyl, 2- oder 3-(Imidazol-1-yl)propyl, Cyclopentyl, Cyclohexyl, Cycloheptyl, 3-Aminomethyl-3,5,5-trimethylcyclohexyl, 2,2,6,6-Tetramethylpiperidin-4-yl, 3,6,9-Trioxadodecyl, 3,6,9,12-Tetraoxatridecyl, 3,6,9,12-Tetraoxatetradecyl, 5-Hydroxy-3-azapentyl, 5-Hydroxy-3-(2-hydroxethyl)-2-methyl-3-azapentyl, 5-Hydroxy-2-methyl-3-azapentyl, 5-Hydroxy-3-(2-hydroxyethyl)-2-methyl-3-azapentyl, 8-Hydroxy-2-methyl-3-aza-6-oxaoctyl, 11-Hydroxy-2-methyl-3-aza-6,9-dioxaundecyl, 8-Hydroxy-3-(5-hydroxy-3-oxapentyl)-2-methyl-3-aza-6-oxaoctyloder 11-Hydroxy-3-(8-hydroxy-3,6-dioxaoctyl)-2-methyl-3-aza-6,9-dioxaundecyl.

Reste $R^3$ und $R^4$ sind weiterhin z.B. Methoxy, Ethoxy, Propoxy, Isopropoxy, Butoxy, Isobutoxy, sec-Butoxy, Fluor, Chlor oder Brom.

Reste $R^3$ sind weiterhin z.B. Acetylamino, Propionylamino, Butyrylamino, Isobutyrylamino, Pentanoylamino, Phenylazo oder 2-, 3- oder 4-Methylphenylazo.

Reste $L^1$, $L^2$ und $L^3$ sind z.B. Ethylen oder 1,2- oder 1,3-Propylen.

Reste $L^1$ sind weiterhin z.B. Chlorethylen oder Chlor-1,2- oder 1,3-propylen.

Reste $L^2$ sind weiterhin z.B. Methylen.

Reste $L^3$ sind z.B. 1,2-, 2,3- oder 1,4-Butylen.

Das Äquivalent $An^{\ominus}$ eines Anions leitet sich z.B. von folgenden Anionen ab: Fluorid, Chlorid, Bromid, Iodid, Phosphat, Hydrogenphosphat, Dihydrogenphosphat, Formiat, Acetat, Propionat, Mono-, Di- oder Trichloracetat, Lactat, Methoxyacetat, Citrat, Succinat, Methansulfonat, Benzolsulfonat, 2- oder 4-Methylbenzolsulfonat oder Naphthalinsulfonat.

Bevorzugt sind Farbstoffe der Formel I, in der

$R^1$ Wasserstoff und

$R^2$ $C_1$-$C_{13}$-Alkyl bedeuten, das durch 1 oder 2 Sauerstoffatome in Etherfunktion, Imino- oder $C_1$-$C_4$-Alkyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist.

Bevorzugt sind weiterhin Farbstoffe aus Formel I in der $R^3$ $C_1$-$C_4$-Alkyl, $C_1$-$C_4$-Alkoxy, $C_2$-$C_4$-Alkanoylamino oder Phenylazo und $R^4$ Wasserstoff bedeuten.

Die neuen basischen Azofarbstoffe der Formel I können nach an sich bekannten Methoden erhalten werden.

Beispielsweise kann ein Anilin der Formel III

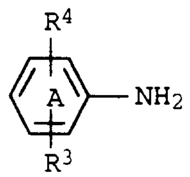

(III),

in der $R^3$, $R^4$ und der Ring A jeweils die obengenannte Bedeutung besitzen, auf an sich bekanntem Weg diazotiert und mit einem Naphthalinderivat der Formel II

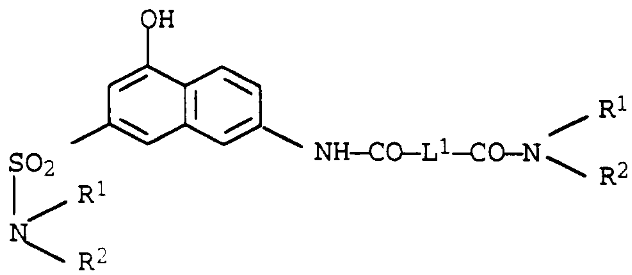

(II),

in der $R^1$, $R^2$, und $L^1$ jeweils die obengenannte Bedeutung besitzen, gekuppelt werden.

Diejenigen Farbstoffe der Formel I, die über ein quartäres Stickstoffatom verfügen, werden zweckmäßig so erhalten, daß man zunächst den neutralen Azofarbstoff herstellt und diesen dann anschließend mit einem $C_2$-$C_4$-Alkylenoxid oder mit einer Verbindung der Formel IV

$Q^4$—Y (IV),

in der $Q^4$ gegebenenfalls durch Hydroxy substituiertes $C_1$-$C_4$-Alkyl und Y eine Austrittsgruppe, z.B. Chlorid, Bromid, Iodid, Methosulfat,-Ethosulfat, Benzolsulfonat oder 2- oder 4-Methylbenzolsulfonat, bedeuten, quaterniert.

Die erfindungsgemäßen basischen Azofarbstoffe der Formel I können für sich alleine, in Gemischen untereinander und zusammen mit anderen kationischen oder anionischen Verbindungen in Form ihrer Lösungen oder in Form von Pulvern oder Granulaten angewendet werden. Sie eignen sich vorteilhaft zum Färben oder Bedrucken von polymerem Material, insbesondere von Papierstoffen, aber auch von Cellulose, Baumwolle, Leder, Bastfasern, Hanf, Flachs, Sisal, Jute, Kokos oder Stroh.

Bei der Herstellung von Farbstoffpräparationen, enthaltend die neuen Farbstoffe der Formel I, ist die Verwendung von Polymeren, wie Polyacrylsäuren, Polyacrylsäurederivaten, Polyvinylaminen, Polyvinylamiden, Polyvinylacetaten, Polyvinylalkoholen, Polyvinylpyrrolidonen, Polysiloxanen oder Copolymeren der jeweiligen Monomeren hervorzuheben. Desgleichen können Oligomere des Ethylenimins, Ethylenoxids oder Propylenoxids oder Derivate dieser Oligomeren zur Anwendung kommen. Von großer Bedeutung sind auch N-alkylierte Pyrrolidone oder N-(2-Hydroxyethyl)pyrrolidone.

Die Farbstoffe können vorzugsweise bei der Herstellung von in der Masse gefärbtem, geleimtem und ungeleimtem Papier verwendet werden. Sie können ebenfalls zum Färben von Papier nach dem Tauchverfahren angewendet werden.

Das Färben von Papier, Leder oder Cellulose erfolgt nach an sich bekannten Methoden.

Die neuen Farbstoffe oder ihre Präparationen färben das Abwasser bei der Papierherstellung praktisch gar nicht oder nur wenig an, was für die Reinhaltung der Gewässer besonders günstig ist. Sie sind hoch substantiv, melieren nicht, wenn sie auf Papier gefärbt sind und sind weitgehend pH-unempfindlich. Die Färbungen auf Papier zeichnen sich durch eine gute Lichtechtheit aus. Nach längerem Belichten ändert sich die Nuance Ton-in-Ton. Außerdem weisen die erfindungsgemäßen Farbstoffe eine gute Löslichkeit auf.

Die gefärbten Papiere, die eine gute Bleichbarkeit aufweisen, sind naßecht, nicht nur gegen Wasser, sondern ebenfalls gegen Milch, Seifenwasser, Natriumchloridlösungen, Fruchtsäfte oder gesüßte Mineralwasser und wegen ihrer guten Alkoholechtheit auch gegen alkoholische Getränke beständig.

Mit den neuen Farbstoffen kann man auch Polyacrylnitriltextilien oder durch anionische Gruppen modifizierte Polyamid- oder Polyestertextilien färben, foulardieren oder bedrucken.

Die vorliegende Erfindung betrifft weiterhin neue Sulfonamide der Formel II

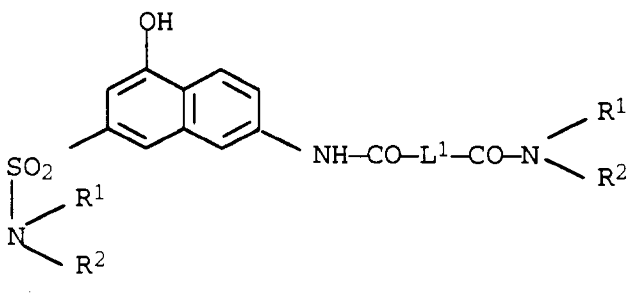
(II),

in der

$L^1$ $C_2$-$C_3$-Alkylen, das gegebenenfalls durch Chlor substituiert ist, Vinylen oder 1,2-Phenylen und

$R^1$ und $R^2$ gleich oder verschieden sind und unabhängig voneinander jeweils $C_1$-$C_{13}$-Alkyl, das gegebenenfalls durch 1 bis 4 Sauerstoffatome in Etherfunktion oder 1 bis 4 Iminogruppen, die durch $C_1$-$C_4$-Alkyl oder $\omega$-Hydroxy-$C_1$-$C_8$-alkyl, das gegebenenfalls durch 1 bis 3 Sauerstoffatome in Etherfunktion unterbrochen ist, substituiert sein können, unterbrochen ist und durch Hydroxy, Amino, einen 5- oder 6-gliedrigen gesättigten oder ungesättigten heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes $C_5$-$C_7$-Cycloalkyl substituiert sein kann, gegebenenfalls substituiertes Phenyl, gegebenenfalls substituiertes $C_5$-$C_7$-Cycloalkyl oder gegebenenfalls durch $C_1$-$C_4$-Alkyl substituiertes Piperidinyl oder $R^1$ auch Wasserstoff oder $R^1$ und $R^2$ zusammen mit dem sie verbindenden Stickstoffatom einen 5- oder 6-gliedrigen gesättigten heterocyclischen Rest, der gegebenenfalls ein weiteres Heteroatom, ausgewählt aus der Gruppe, bestehend aus Sauerstoff und Schwefel, aufweist, bedeuten,

mit der Maßgabe, daß mindestens ein quaternierbares Stickstoffatom im Molekül vorhanden ist.

Bevorzugt sind solche Sulfonamide der Formel II, in der $R^1$ Wasserstoff und $R^2$ $C_1$-$C_{13}$-Alkyl, das mindestens ein quaternierbares Stickstoffatom aufweist, bedeuten.

Die neuen Sulfonamide der Formel II können beispielsweise erhalten werden, indem man I-Säure mit cyclischen Carbonsäureanhydriden der Formel V

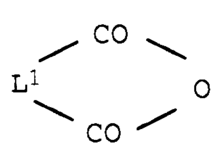
(V),

in der $L^1$ die obengenannte Bedeutung besitzt, umsetzt, das resultierende Reaktionsgemisch zur Überführung der Sulfonsäuregruppe in eine Sulfohalogenidgruppe, z.B. eine Sulfochloridgruppe, mit einem entsprechenden Halogenierungsreagenz, z.B. Phosphoroxidtrichlorid, behandelt und anschließend mit einem Amin der Formel VI

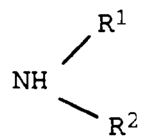

(VI),

in der $R^1$ und $R^2$ jeweils die obengenannte Bedeutung besitzen, zur Reaktion bringt.

Die erfindungsgemäßen Sulfonamide sind wertvolle Zwischenprodukte zur Herstellung der Farbstoffe der Formel I.

Die folgenden Beispiele sollen die Erfindung näher erläutern. Die dort angegebenen Prozente sind Gewichtsprozente.

Beispiel 1

a) 20 g (0,2 mol) Bernsteinsäureanhydrid wurden in 50 g N,N-Dimethylacetamid gelöst und bei 50 °C mit 23,8 g (0,1 mol) I-Säure versetzt. Nach weiteren 1,5 h bei 70 °C wurde auf 30 °C abgekühlt, mit 76,7 g (0,5 mol) Phosphoroxidtrichlorid versetzt und 18 h bei Raumtemperatur gerührt. Die Isolierung des Sulfonsäurechlorids erfolgte durch Hydrolyse in Eiswasser, Absaugen des Feststoffes und anschließendes Aufnehmen in Essigester. Nach Trocknen über Natriumsulfat und Entfernen des Lösungsmittels unter vermindertem Druck resultierten 31,5 g (93 %) Sulfonsäurechlorid.

b) 31,5 g Sulfonsäurechlorid wurden in 270 ml Xylol und 75 ml N-Cyclohexylpyrrolidon gelöst und bei 40 °C zu 35,2 g (0,4 mol) N,N-Dimethyl-1,2-ethylendiamin getropft. Nach weiteren 2 h bei 40 bis 45 °C war die Reaktion beendet. Das Sulfonamid wurde durch Rühren mit 55 g Eisessig und 25 g Wasser in die wäßrige Phase übergeführt und abgetrennt.

c) Die wäßrige Phase wurde bei einem Wert von pH 5 mit 0,08 mol diazotiertem p-Aminoacetanilid versetzt. Der pH-Wert wurde durch Zugabe von wäßriger Natriumcarbonatlösung konstant gehalten.

(Die Lösung des Diazoniumsalzes wurde wie folgt hergestellt: 3,75 g (0,025 mol) p-Aminoacetanilid wurden bei 0 bis 3 °C über 30 min mit 50 ml Wasser und 12,5 ml 30 gew.-%iger Salzsäure gerührt und anschließend mit 65 g Eis versetzt. Die Diazotierung erfolgte durch Zutropfen von 7,5 ml 23 gew.-%iger wäßriger Natriumnitritlösung. Das Reaktionsgemisch wurde weitere 2 h bei 0 bis 3 °C gerührt, klärfiltriert und dann für die Kupplungsreaktion verwendet.)

Durch Aussalzen mit Kochsalz konnte der Farbstoff der Formel

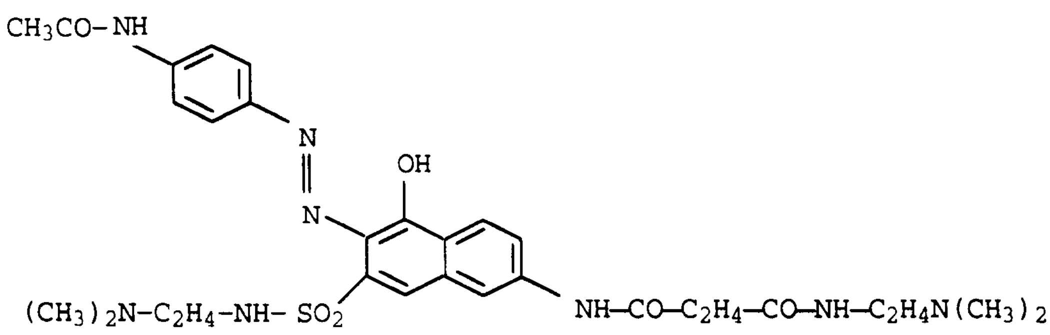

isoliert werden.

λmax: 498 nm (in 30 gew.-%iger Essigsäure)

Beispiel 2

Beispiel 2 wurde analog Beispiel 1 durchgeführt, jedoch wurde das Sulfonsäurechlorid aus Stufe a) bei 20 °C mit 70 g (0,8 mol) N,N-Dimethyl-1,2-ethylendiamin versetzt und 60 h bei Raumtemperatur gerührt. Das resultierende Öl wurde mit 100 ml Wasser versetzt, mit Salzsäure auf einen pH-Wert von 5 gebracht und anschließend, wie unter 1 c) beschrieben, weiter umgesetzt.

In analoger Weise werden die in der folgenden Tabelle aufgeführten Farbstoffe der Formel

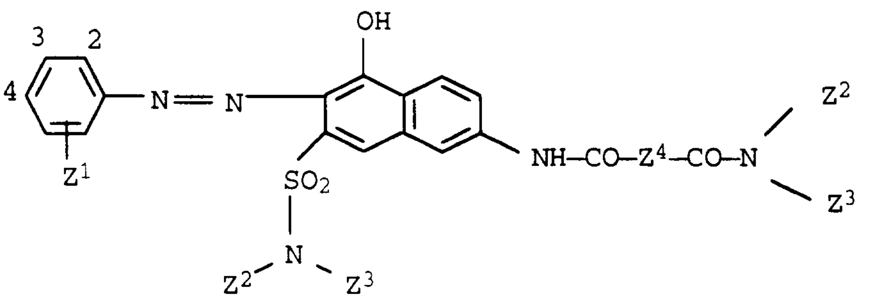

erhalten. (In der 3. Spalte ist jeweils das bei der Reaktion verwendete Amin $HNZ^2Z^3$ angegeben.)

Tabelle

| Bsp. Nr. | $Z^1$ | $HNZ^2Z^3$ | $Z^4$ | $\lambda_{max}$ [nm] * |
|---|---|---|---|---|
| 3 | 4-$NHCOCH_3$ | $H_2N(CH_2)_3N(C_2H_5)_2$ | $CH_2CH_2$ | 498 |
| 4 | 4-$NHCOCH_3$ | $H_2N(CH_2)_3NH(CH_2)_3NH_2$ | $CH_2CH_2$ | 498 |
| 5 | 4-$NHCOCH_3$ | 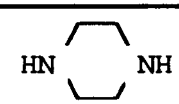 | $CH_2CH_2$ | 500 |
| 6 | 4-$NHCOCH_3$ | $H_2N(CH_2)_2NH_2$ | $CH_2CH_2$ | 496 |
| 7 | 4-$NHCOCH_3$ | $H_2N(CH_2)_3NH_2$ | $CH_2CH_2$ | 498 |
| 8 | 4-$NHCOCH_3$ | $H_2N(CH_2)_4NH_2$ | $CH_2CH_2$ | 498 |
| 9 | 2-$OCH_3$ | $H_2N(CH_2)_2NH_2$ | $CH_2CH_2$ | 498 |
| 10 | 2-$OCH_3$ | $H_2N(CH_2)_3NH_2$ | $CH_2CH_2$ | 496 |
| 11 | 2-$OCH_3$ | $H_2N(CH_2)_4NH_2$ | $CH_2CH_2$ | 500 |
| 12 | 2-$OCH_3$ | $H_2N(CH_2)_3NH(CH_2)_3NH_2$ | $CH_2CH_2$ | 498 |
| 13 | 4-$NHCOCH_3$ | $H_2NCH(CH_3)CH_2NH_2$ | $CH_2CH_2$ | 498 |

*) gemessen in 30 gew.-%iger Essigsäure

## Patentansprüche

1. Farbstoffe der Formel I

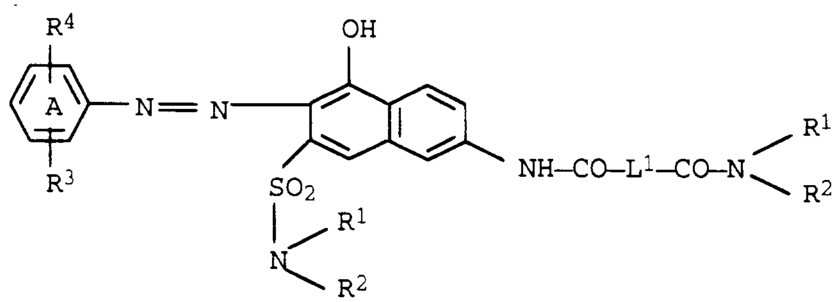

(I),

in der
der Ring A benzoanelliert sein kann,

| | |
|---|---|
| $L^1$ | $C_2$-$C_3$-Alkylen, der gegebenenfalls durch Chlor substituiert ist, Vinylen oder 1,2-Phenylen, |
| $R^1$ und $R^2$ | gleich oder verschieden sind und unabhängig voneinander jeweils $C_1$-$C_{13}$-Alkyl, das gegebenenfalls durch 1 bis 4 Sauerstoffatome in Etherfunktion oder 1 bis 4 Iminogruppen, die durch $C_1$-$C_4$-Alkyl oder $\omega$-Hydroxy-$C_1$-$C_8$-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder ungesättigten heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls sübstituiertes $C_5$-$C_7$-Cycloalkyl sübstituiert sein kann, gegebenenfalls substituiertes Phenyl, gegebenenfalls substituiertes $C_5$-$C_7$-Cycloalkyl oder gegebenenfalls durch $C_1$-$C_4$-Alkyl substituiertes Piperidinyl oder $R^1$ auch Wasserstoff oder $R^1$ und $R^2$ zusammen mit dem sie verbindenden Stickstoffatom einen 5- oder 6-gliedrigen gesättigten heterocyclischen Rest, der gegebenenfalls ein weiteres Heteroatom, ausgewählt aus der Gruppe, bestehend aus Sauerstoff und Schwefel, aufweist, |
| $R^3$ | Wasserstoff, $C_1$-$C_4$-Alkyl, $C_1$-$C_4$-Alkoxy, $C_2$-$C_5$-Alkanoylamino, Halogen, Nitro, Cyano, gegebenenfalls durch $C_1$-$C_4$-Alkyl substituiertes Phenylazo oder einen Rest der Formel |

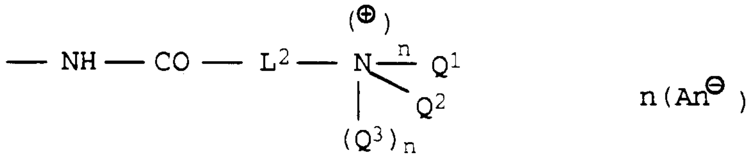,

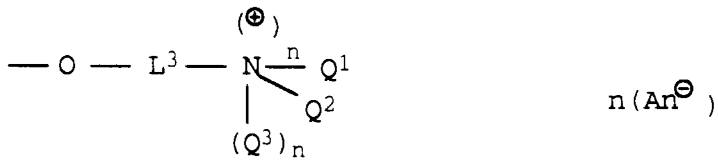,

oder

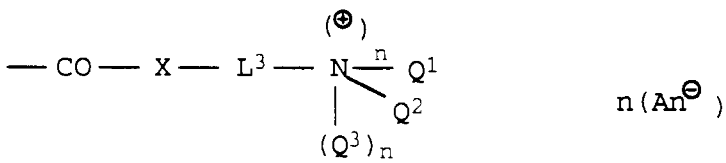,

worin X für Sauerstoff oder Imino,

$L^2$ für $C_1$-$C_3$-Alkylen,

$L^3$ für $C_2$-$C_4$-Alkylen,

n für 0 oder 1,

$Q^1$, $Q^2$, $Q^3$ gleich oder verschieden sind und unabhängig voneinander jeweils für Wasserstoff oder gegebenenfalls durch Hydroxy substituiertes $C_1$-$C_8$-Alkyl, das durch 1 bis 3 Sauerstoffatome in Etherfunktion oder 1 bis 3 Iminogruppen, die durch $C_1$-$C_4$-Alkyl oder ω-Hydroxy-$C_1$-$C_8$-alkyl, das gegebenenfalls durch 1 bis 3 Sauerstoffatome in Etherfunktion unterbrochen ist, substituiert sein können, unterbrochen sein kann, oder $Q^1$ und $Q^2$ zusammen mit dem sie verbindenden Stickstoffatom für einen 5- oder 6-gliedrigen gesättigten oder ungesättigten heterocyclischen Rest, der gegebenenfalls ein weiteres Heteroatom, ausgewählt aus der Gruppe, bestehend aus Stickstoff, Sauerstoff und Schwefel, aufweist, und

$An^{\ominus}$ für das Äquivalent eines Anions stehen, und

$R^4$ Wasserstoff, $C_1$-$C_4$-Alkyl, $C_1$-$C_4$-Alkoxy, Halogen, Nitro oder Cyano bedeuten,

mit der Maßgabe, daß mindestens ein quaternierbares oder quartäres Stickstoffatom im Molekül vorhanden ist.

2. Farbstoffe nach Anspruch 1, dadurch gekennzeichnet, daß

$R^1$ Wasserstoff und

$R^2$ $C_1$-$C_{13}$-Alkyl bedeuten, das durch 1 oder 2 Sauerstoffatome in Etherfunktion, Imino- oder $C_1$-$C_4$-Alkyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist.

3. Farbstoffe nach Anspruch 1, dadurch gekennzeichnet, daß

$R^3$ $C_1$-$C_4$-Alkyl, $C_1$-$C_4$-Alkoxy, $C_2$-$C_4$-Alkanoylamino, oder Phenylazo und

$R^4$ Wasserstoff bedeuten.

4. Verwendung der Farbstoffe gemäß Anspruch 1 zum Färben oder Bedrucken von polymerem Material.

**5.** Sulfonamide der Formel II

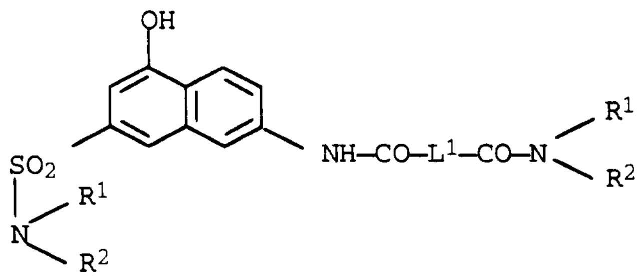

(II),

in der

$L^1$ $C_2$-$C_3$-Alkylen, das gegebenenfalls durch Chlor substituiert ist, Vinylen oder 1,2-Phenylen und

$R^1$ und $R^2$ gleich oder verschieden sind und unabhängig voneinander jeweils $C_1$-$C_{13}$-Alkyl, das gegebenenfalls durch 1 bis 4 Sauerstoffatome in Etherfunktion oder 1 bis 4 Iminogruppen, die durch $C_1$-$C_4$-Alkyl oder ω-Hydroxy-$C_1$-$C_8$-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder ungesättigten heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes $C_5$-$C_7$-Cycloalkyl substituiert sein kann, gegebenenfalls substituiertes Phenyl, gegebenenfalls substituiertes $C_5$-$C_7$-Cycloalkyl oder gegebenenfalls durch $C_1$-$C_4$-Alkyl substituiertes Piperidinyl oder $R^1$ auch Wasserstoff oder $R^1$ und $R^2$ zusammen mit dem sie verbindenden Stickstoffatom einen 5- oder 6-gliedrigen gesättigten heterocyclischen Rest, der gegebenenfalls ein weiteres Heteroatom, ausgewählt aus der Gruppe, bestehend aus Sauerstoff und Schwefel, aufweist, bedeuten,

mit der Maßgabe, daß mindestens ein quaternierbares Stickstoffatom im Molekül vorhanden ist.